# EUROPEAN PATENT SPECIFICATION

(11) **EP 2 491 161 B1**
(45) Date of publication and mention of the grant of the patent: **16.04.2014**
(21) Application number: 11705583.0
(22) Date of filing: 28.02.2011
(51) Int. Cl.: C23C 14/02, C23C 14/06, A61L 27/30, A61L 31/08, C23C 14/32, C23C 14/35

(54) **SURFACE COATINGS FOR MEDICAL IMPLANTS**
OBERFLÄCHENBESCHICHTUNGEN FÜR MEDIZINISCHE IMPLANTATE
REVÊTEMENTS DE SURFACE POUR DES IMPLANTS MÉDICAUX

(30) Priority: 26.02.2010 EP 10154838
(43) Date of publication of application: 29.08.2012
(73) Proprietor: IMC Technology Consultants GmbH, 1010 Wien (AT)
(72) Inventor: LAMMER, Johannes, CH-6535 Roveredo (CH)
(74) Representative: Sonn & Partner Patentanwälte
(86) International application number: PCT/EP2011/052924
(87) International publication number: WO 2011/104384

(56) References cited:
- WO-A1-2006/067031
- US-A1- 2009 120 241
- US-A1- 2009 324 937
- WEN L S ET AL: "A transmission electron microscopy study on Ti-N films deposited by ion plating", JOURNAL OF VACUUM SCIENCE & TECHNOLOGY A (VACUUM, SURFACES, AND FILMS) USA, vol. 4, no. 6, November 1986 (1986-11), pages 2682-2685, XP002590053, ISSN: 0734-2101

## Description

The present invention relates to surface coatings usable for medical implants and micromechanical devices.

Medical implants such as orthopaedic or cardiovascular implants, are usually metallic alloys, ceramics and composites formed from biocompatible polymers and various reinforcing materials.

Metals and metal alloys such as stainless steel, titanium and titanium alloys have been used successfully for many years as implant materials, particularly for orthopaedic applications. These materials have the requisite strength characteristics needed for such implants but are susceptible to fretting, wear and corrosion in the body unless treated to reduce these effects. Particulates produced by joint articulation processes or micro motion between assembled devices tend to cause accelerated wear of prosthetic joints and trauma devices.

Further, concern has been raised about potential abrasion between implant metals and adjacent bone and bone cement. This abrasion creates particulates, which are associated with adverse cellular response, including bone cell death and eventual loosening of the implant and subsequent revision.

A major problem of implants known in the art, in particular of deformable implants such as stents, is that the biocompatible coating of such implants, when deformed or subjected to physical stress, tends to chip off. In order to avoid this problem several solutions are suggested in the art.

WO 2006/067031, for instance, relates to implants being coated with a non-porous barrier layer consisting essentially of an essentially stoichiometric titanium nitride layer. The coating described therein suffers several shortcomings. In particular the question of adhesion is object of multiple statistic variations, thus necessitating extensive quality management tools, increasing by this unduly the cost of production. The multilayer coating according to WO 2006/067031 contains an elevated number of interfaces between the functional layers, which increases the probability of failure (scaling) of the coating. The main drawback of these coatings resides in the fact that it contains six or even 12 interfaces.

It is an object of the present invention to provide methods and means to overcome the drawbacks of the state of the art.

The present invention relates to a substrate comprising a single layer coating comprising an ion-implanted adhesion area (1, 2) located on the inner side of the layer, a cross-sectional area (3, 4, 5a, 5b, 6, 7, 8, 9) being characterised by a variation of its composition within its cross-section sandwiched between said adhesion area and an outermost area (10, 11), wherein
- the ion-implanted adhesion area being composed of titanium, zirconium, hafnium or alloys thereof (1, 2) has a thickness of 0.1 to 10 nm,
- the cross-sectional area (3, 4, 5a, 5b, 6, 7, 8, 9) comprises nitrides of titanium, zirconium, hafnium or alloys thereof having the general formula M_{X}N_{Y} with X+Y being 1 and X<1, 0<Y<1, with M being titanium, zirconium, hafnium or alloys thereof and with N being nitrogen, and having a thickness of 2 to 30 nm, preferably of 2 to 20 nm, in which is contained an area (5b) of a composition of M_{X}N_{Y1} with Y1 being a number between 0.9 and 1.1 having throughout its cross-section a nitrogen gradient between 1 and 100 %, and
- the outermost area (10, 11) comprises oxynitride of the general formula MN_{Z}O_{W}, wherein Z+W is 1, with Z varying from 0.2 to 0.9, with M being a solid transition metal of group IV B, V B or VI B of the periodic table of the elements or a solid solution thereof, with N being nitrogen and with O being oxygen, and has a thickness of 1 to 200 nm, preferably 2 to 50 nm.

It surprisingly turned out that a coating comprising M_{X}N_{Y} on a deformable substrate is much more stable against chipping off of the coating when subjected to extreme strain and stress. Such a coating can be obtained, for instance, with the method described below.

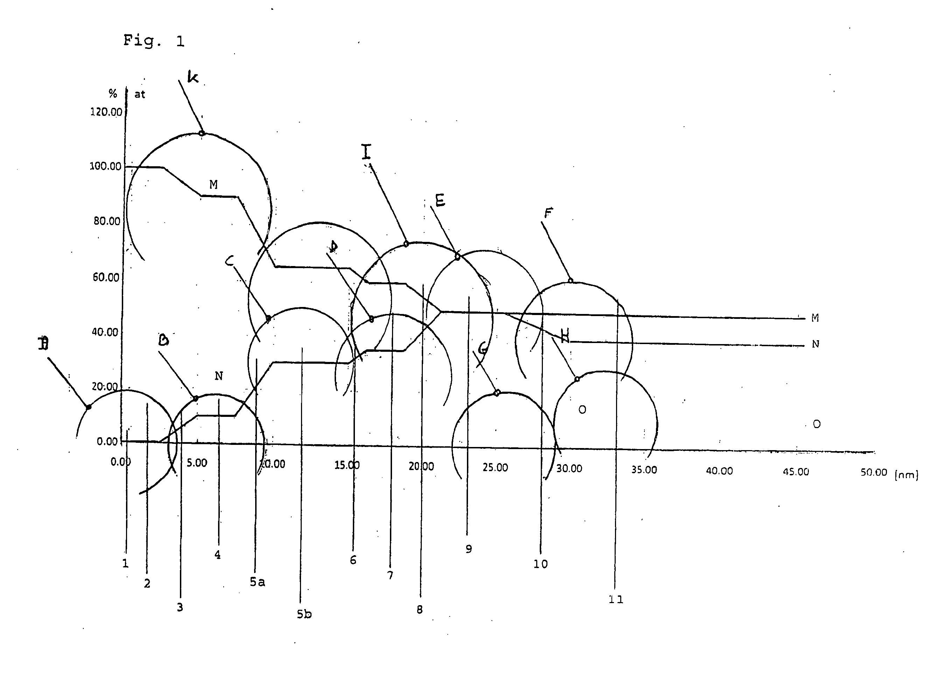

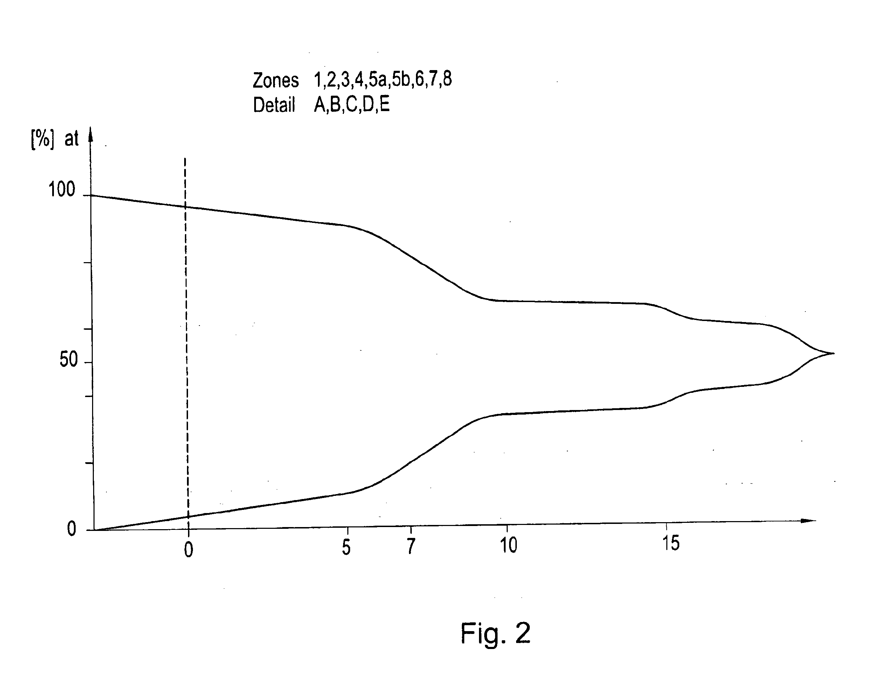

The present invention relates to a single layer ceramic coating comprising different nitrogen content regions. In particular it contains a region of composition close to M_{X}N_{Y} with X being preferably 2 and Y being 0.9 to 1, preferably 0.95 to 1.05. Said one region being 0.1 to 25 nm, preferably 5 nm, thick (see e.g. Fig. 1 and Fig. 2). It shall be noted that the gradient of nitrogen variation shall never be lower than 5 %.

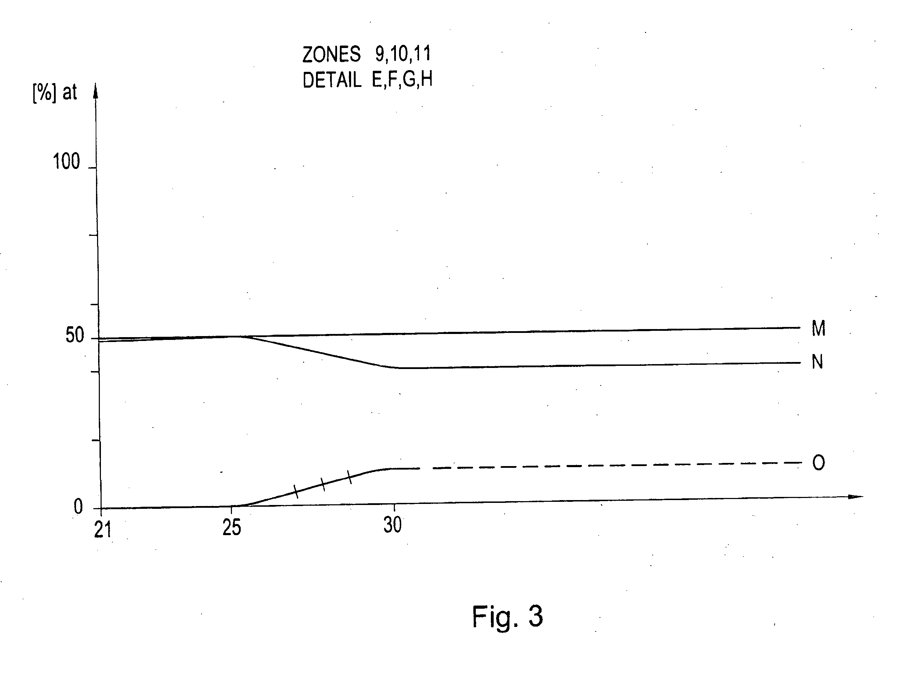

The various cross-sectional areas of the coating layer of the present invention are stoichiometrically non-homogeneous (see Fig. 1). Furthermore the areas between the various areas represent continuous nanometric variations in stoichiometry. The composition of area 5b and 9 may be M_{X}N_{Y}, M₂N and MN, respectively, with N being close to 1. M may be titanium which can be replaced by a solid solution of metals of the IV B, V B or VI B elements of the periodic table of elements, in particular solid solutions of 80 % of Ti with remainder being zirconium and hafnium. The solid solutions such defined will be designated as M. Such the nonstoechiometric composition will be M₂Nx (X = 0.95 to 1.05) and MNx with x proportions as before. The coating region of the top layer is being coated with a compound MN_{Z}O_{A} (Metal-Oxy-Nitride), with a preferred nitrogen/oxygen ratio of 4:1 (see Fig. 1 and Fig. 3).

The coating of the present invention can be suitably used for deformable substrates.

The solid transition metal of group IV B, V B or VI B of the periodic table of the elements are Ti, V, Cr, Zr, Nb, Mo, Hf, Ta and W.

"Deformable", as used herein, refers to materials and implants whose structure can be reversibly deformed. Examples for deformable medical implants are, for instance, stents.

In a particularly preferred embodiment of the present invention the substrate material is coated with one single layer. The cross section of said single layer may be divided into various areas or stretches surrounding the substrate material having varying compositions. At least one of these areas or stretches comprises or contains M₂N, preferably Ti₂N.

The part of the single layer adjacent to the surface of a substrate, the adhesion area, is composed mainly of titanium, zirconium, hafnium or alloys thereof. This part of the single layer is responsible for the adhesion of said single layer to the surface of the substrate material. Furthermore this part may have a thickness of 0.5 to 15 nm, preferably 0.1 to 10 nm or 1 to 10 nm, more preferably 1 to 5 nm or 2 to 5 nm, even more preferably 1 to 3 nm. A further area of the single layer may comprise nitrides of titanium, zirconium, hafnium or alloys thereof, such as TiN or Ti₂N more generally M₂N and MN, respectively. The titanium alloy preferably comprises 20 % zirconium or hafnium. These areas or stretches have a thickness of 2 to 30 nm, preferably 2 to 20 nm.

Those areas of the single layer which are exposed to the human or animal tissue and have to be biocompatible, the outermost area, comprise preferably oxynitrides of titanium, zirconium, hafnium or alloys thereof. These areas or stretches have a thickness of 1 to 200 nm, preferably of 2 to 50 nm, more preferably of 0.1 to 50 nm, even more preferably of 0.1 to 25 nm or 2 to 30 nm, most preferably of 2 to 20 nm.

In order to obtain such a single layer physical vapour deposition may be used. The single layer is formed by varying the gas mixture within the device. The addition of N₂ to the inert gas (e.g. Ar) within the vapour deposition device results in the deposition of nitrides of the target metal. The addition of O₂ to the inert gas results in the formation of oxides. Adding N₂ as well as O₂ to the vacuum chamber of the vapour deposition device results in the formation of oxynitrides of the target metal or target alloy.

According to a preferred embodiment of the present invention the coating layer further comprises oxides, nitrides, oxynitrides, carbides and/or borides of titanium, hafnium and/or zirconium or more generally the solid solutions of the metallic elements of group VI B, V B and VI B of the periodic table of the elements.

According to a further preferred embodiment of the present invention the oxides, nitrides, oxynitrides, carbides and/or borides are selected from the group consisting of titanium nitride (TiN), titanium oxynitride etc..

The coating layer of the present invention can be considered as "single layer coating nanostructured in stoichiometry". This means that the various areas of the coating layer have a variable, modulated stoichiometry in their cross section.

The deformable medical implant according to the present invention has to be biocompatible. In order to obtain a biocompatible surface the single layer of the present invention comprises or consists of an oxynitride, which is preferably titanium oxynitride having the formula TiN_{A}O_{B}, wherein A+B = 1, with A varying from 0.1 to 0.9, preferably from 0.2 to 0.5, particularly being 0.25.

The use of a coating layer according to the present invention has the advantage that already with one coating layer the inventive effect is achieved. Consequently, the at least one coating layer has preferably a thickness of 10 to 200 nm, preferably 20 to 100 nm, more preferably 30 to 60 nm, in particular 50 nm.

According to a preferred embodiment of the present invention an adhesion area is provided between the substrate and the other areas of the coating layer. The adhesion area, as discussed above, is preferably an integral part of the coating layer.

The solid transition metal of the adhesion area is preferably zirconium, titanium or hafnium or a combination thereof with a nitrogen gradient of 5% (see Fig. 2).

According to a preferred embodiment of the present invention the adhesion area has a thickness of 0.5 to 15 nm, preferably 1 to 10 nm, more preferably 2 to 5 nm. The thickness of the adhesion area depends on the energy of the impinging particles, being in the order of 30 to 200 eV.

In order to obtain a coating layer with the desired prosperties the area comprising M_{X}N_{Y}, preferably double titanium nitride (Ti₂N), has a thickness of 0.2 to 20 nm, preferably 0.5 to 15 nm, more preferably 1 to 10 nm.

The substrate can be of any metallic material, whereby it is preferred that the substrate material is selected from the group of steel, nitinol, titanium or alloys thereof as well as cobalttitanium-nickel and Co-Cr-Mo alloys.

The physical and biochemical properties of the single layer coatings according to the present invention allow them to be used on implants. According to a preferred embodiment of the present invention the implant is an orthopaedic implant, preferably a hip, knee, shoulder or elbow implant, or a vascular implant, preferably a vascular graft or a vascular stent.

Implants, in particular stents, being in contact with the human body or with the bloodstream should be biocompatible, in particular should exhibit anti-thrombogenic functions and favour endothelization at the outside boundary of the stent. TiN or TiN_{A}-O_{B} on the surface of the substrate or medical implant favours these properties.

Another aspect of the present invention relates to a method for producing a coating layer of a substrate according to the present invention using a deposition apparatus comprising a vacuum chamber and a power supply having controllable supply parameters, in which the substrate is enclosed in the vacuum chamber and subjected to bombardment by electrically-excited ions by electrical excitation provided by the power supply, said method comprising the steps of:
a) providing an inert gas and a target comprising titanium, chromium, zirconium and hafnium or alloys thereof, in the vacuum chamber,
b) performing a physical vapour deposition process for coating the surface of the substrate with an adhesion layer comprising titanium, chromium, zirconium and hafnium or alloys thereof, and
c) introducing into the vacuum chamber a gas mixture comprising a varying amount of nitrogen and/or oxygen while performing the physical vapour deposition process, wherein step c) is performed in part with a target consisting of titanium, zirconium or alloys thereof in an atmosphere containing 10 to 30% N₂.

The deposition apparatus according to the present invention can be anyone known in the art.

In contrast to usual methods for depositing coatings on a surface the method according to the present invention involves the use of a gas mixture whose composition changes throughout the process. This allows varying the composition of the layer during the deposition process.

Furthermore, the cross section of a layer obtainable by this method, wherein the composition of the gas mixture is changed, does not comprise interfaces between the various areas. The lack of interfaces between the areas of the coating layer is - next to the provision of at least one area comprising double titanium nitride (Ti₂N) - responsible for the superior properties of the coating layer so that the layer on the substrate does not scale off when exposed to mechanical stress.

In order to obtain a good adhesion penetration of the area of the coating layer adjacent to the surface of the substrate (see also area 1 in Fig. 1) the preferred deposition methods may preferably involve high power magnetron sputtering or alternatively, for even higher particle penetration, filtered arc deposition, which can be improved by Wehnelt cylinder energy controlling techniques.

PVD procedures are well established coating procedures for electric and electronic devices. Standard sputtering and magnetron sputtering, however, suffer from the fact, that the primary particle energy is limited to approximatively 12 eV, thus not allowing the penetration of the sputtered particles into the substrate, with following relatively reduced adhesion, following the laws of van der Waal. This weakness was overcome, for instance, by WO 2006/067031 A1 in applying up to nine subsequent coatings of constant stoichiometry in order to compensate for internal stresses. However, as discussed above, also this solution is not satisfactory. Therefore it is preferred to apply high energy radio-frequency magnetron sputtering with resulting particle energies of up to 30 eV enabling thus ion-implantation of the sputtered ions to a depth of at least 1 nanometres (nm) as shown, for instance, in Fig. 1.

Arc deposition yields primary particle intensities of approximatively 200 eV. The ion-beam energy distribution can be uniformed by the well known Wehnelt-cylinder technique to a span of +/- 5 %. Furthermore the particles of this ion-beam, uniform in energy, can easily be accelerated to up to 1000 eV. This acceleration as well as the separation of the ions and the neutrals can be done with techniques, which are the state of the art, by electromagnetic or electrostatic fields (e.g. US 2002/007796). This variation of the particle beam intensities permits tailoring of the particle implantation into the substrate, optimizing the adhesion as a function of the substrate material and the coating material selected. The multiple coating approach can thus be essentially simplified. The addition of reactive gases in variable proportions permits the nanostructuring of the stoichiometry of the single layer in very close ranges, assuring such complete quality management of the total layer and by mastering the primary energy of the impinging particles to absolutely control the penetration depths of the aforementioned coating material and consequently completely master the critical parameter of each coating i.e. adhesion.

With the method of the present invention the modulus of Young E and the critical stress intensity factor Kic across the section of the layer can be modulated. This modulation allows obtaining a coating layer with the claimed properties.

According to a preferred embodiment of the present invention the physical vapour process is selected from the group of sputter deposition, cathodic arc deposition, reactive filtered arc plasma deposition, reactive filtered arc plasma deposition with Wehnelt plasma confinement, reactive filtered arc plasma deposition with Wehnelt plasma confinement and an electrostatic or electromagnetic scanning device and high intensity radio frequency magnetron sputtering.

In order to produce a coating as described herein the amount of nitrogen and/or oxygen in the gas mixture of step c) is stepwise or gradually increased from 0 to 30 %, preferably from 0 to 20 %, more preferably from 0 to 15 %, while performing the physical vapour deposition process to obtain a coating layer with a thickness of 10 to 200 nm, preferably 20 to 100 nm, more preferably 30 to 60 nm, in particular 50 nm.

In order to obtain the desired thickness of the various areas of the coating layer the device to perform the physical vapour process is preferably calibrated. The calibration occurs by using RBS analysis (Rutherford-Back-Scattering) which allows determining the composition as well as the deposition rate thus obtaining a time-gas flow diagram. This diagram is used afterwards to coat the substrates of the present invention.

According to a further preferred embodiment of the present invention the amount of nitrogen in the gas mixture is stepwise or gradually increased to 30 %, preferably 25 %, more preferably to 20 %, followed by a stepwise or gradual increase of oxygen in the gas mixture to 20 %, preferably 10 %.

It is particularly preferred that the ratio N₂/O₂ is about 3/1 to 5/1, preferably about 4/1.

According to a further preferred embodiment of the present invention the substrate is an implant which is preferably an orthopaedic implant, preferably a hip, knee, shoulder or elbow implant, or a vascular implant, preferably a vascular graft or a vascular stent.

According to a particularly preferred embodiment of the present invention the coating layer comprises or consists of the following cross-sectional areas (from the surface of the substrate to the surface of the coating layer): an adhesion area having a thickness of 0.5 to 15 nm, preferably 1 to 10 nm, more preferably 2 to 5 nm, being composed of a solid transition metal of group IV B, V B or VI B of the periodic table of the elements or a combination thereof, preferably titanium, chromium and/or zirconium; at least one area of nitrides of at least one solid transition metal of group IV B, V B or VI B of the periodic table of the elements or solid solutions thereof, preferably titanium, hafnium and/or zirconium with a thickness of 0.1 nm to 30 nm, preferably 0.5 to 20 nm; one area comprising double titanium nitride (Ti₂N), said one area being 0.1 to 25 nm thick; an area comprising or consisting of titanium oxynitride has the formula TiN_{A}O_{B}, wherein A+B = 1, with A varying from 0.1 to 0.9, preferably from 0.2 to 0.5, particularly being 0.25.

As disclosed above the substrates to be coated with the method according to the present invention are preferably medical implants, such as stents.

An example of preferred stents is intraluminal coronary ballooning stents.

Intraluminal coronary ballooning stents represent one of the major progresses in non intrusive surgery. However, the mechanical properties of aforesaid stents have to combine a multitude of sometimes contradictory properties. On the one side they have to be very flexible in order to being introduced into the intricated body blood vessel system, they have to show appropriate stiffness in order to resist to buckling during stenting, during ballooning they will be submitted to a radial extension of at least 200 % and at the same time will be submitted to a longitudinal shrinkage of about 10 %. The ceramic coating according to the present invention may undergo a 400 % radial expansion free of fissures and any scaling. Any of the claimed combinations of ceramic coatings, according to the present invention can be applied. The decision upon the right sequence nanostructured stoichiometry are physical, chemical, mechanical, biological and medical. It is important to make the right alterations between Young Modulus and critical stress intensity factor Kic, ultimate tensile strength and maximal elongation before rupture. A stent coated this way may expect a useful lifetime of 20 years. The coating would be preferentially performed by reactive filtered Arc-ion beam deposition and high power reactive radio-frequency magnetron sputtering according to the present invention.

The tribiological problem of hip and knee implants is still not solved to full satisfaction. The very low coefficient of friction of titanium nitride combined with the excellent cohesion and strain-stress properties and fatigue properties of the claimed single layer coating nanostructured in its stoichiometry will provide a new solution to this important problem.

The flexible part of pacemaker electrodes can now be coated with efficient protection against Staphylococcus and bacteriophage.

Syringes for medium and long term application in the human body can be protected by the same principle. Even in case of accidental bending the protective coating will remain well positioned.

Removable electronic hearing aids, subject to multiple human intervention and thus subject to many infections, can be positively protected. All of these coatings can be performed according to the present invention and be optimized with regard to the physico-chemical requirements of the embodiments.

The coating according to the present invention may also be applied on mechanical watch regulatory mechanisms.

The regulatory mechanical watch mechanism, anchor and escape wheel, are amongst the most solicitated mechanical parts known to engineers. An important increase in lifetime or decrease in the maintenance cycle can be obtained if these mechanical parts could be coated with reliable, fatigue proof coating. A single layer coating nanostructured in its stoichiometry composed of an implantation layer of chromium and titanium followed of carbonitride of titanium or titaniumaluminide and a final stoichiometry of titanium nitride according to the present invention may be used. The anchor could be coated with a single-layer coating nanostructured in its stoichiometry of titanium or chromium or both, followed by a sandwich consisting of titaniumnitride, titaniumcarbide and diamond like carbon (DLC) with continous varying stoichiometry assuring thus a maintenance free timekeeping mechanism.

The present invention is further illustrated by the following figures and by the following example, however, without being restricted thereto.

Fig. 1 shows the composition of the different areas of the single layer coating in relation to the thickness of the coating layer obtainable by the method according to the present invention. M is a metal as defined herein, N is nitrogen and O is oxygen.

The adhesion area 1 and 2 is composed of a solid transition metal of group IV B, V B or VI B of the periodic table of the elements. The thickness of this adhesion area in Fig. 1 is about 2.5. The cross-sectional area 3, 4, 5a, 5b, 6, 7, 8 and 9 comprises metal nitrides in various molar ratios. The addition of oxygen in the course of the manufacture results in the formation of oxynitrides forming the outermost area 10 and 11 of the coating.

Fig. 2 and 3 are to highlight the continuous variation of the stoichiometry of the single layer, thus producing the desired variation of the critical stress coefficient. The top coating, however, is stabilizing towards a constant value, dictated by the desired mechanical, chemical, tribological or medical requirements.

It is well put into evidence that the single layer coating can be considered as a gradient alloy.

### EXAMPLE:

The person skilled in the art understands that the parameters given in the following example can be influenced by the parameters of the existing coating equipment, such as coating chamber geometry, pumping equipment and baffle facilities, thus influencing the residence time of the reactive species in the coating area.

A single layer coating nano-structured in its stoichiometry shall be applied to cardiovascular implants (stents).

After classical ultrasonic cleaning in appropriate aqueous solutions of cleaning agents, after drying, the stents will be placed on the substrate support. The substrate support consists of a vertical structure, submitted to a double planetary movement, allowing the stents to circulate around a central axis and suspended on individual mandrels to rotate around its own axis. 500 stents of a length of 16 mm can thus be exposed uniformly to the flux of the incoming ion-beam. Equally possible is a horizontal sample holder in case of vertical up-down coating. This sample holder would however be in a continuous oscillating horizontal movement with an amplitude of approx. 3.7 times the bare stent diameter and a frequency of approx. 1 Hz. Each stent will in this case be lodged in an individual chamber according to its dimension.

The vacuum chamber was pumped to 10 hPa.

The chamber was filled with Ar to a pressure of 10⁻³ hPa.

The filtered and accelerated arc was ignited. During this time the substrates were protected from the ion-beam by an appropriate shutter.

After stabilisation of the ion-beam, after approx. 2 min., the shutter was opened and the substrates were subjected to a beam of 500 eV, thus creating an ion-implantation and adhesion layer of 1 to 5 nm thickness. This primary coating was performed with pure titanium in the case of coronary implants.

Subsequently the nanometric continuous variation of the partial pressure in the coating chamber in order to achieve the desired chemical and mechanical and furthermore bioactive properties of the coating took place. During the coating of the substrate the argon arc gas was partially substituted by nitrogen by maintaining the total pressure at 10⁻³ hPa. The nitrogen partial pressure was increased continuously until reaching an intermediate area of M₂N and later on stoichiometry of pure titanium nitride. This step of the procedure lasted approx. 5 min. In order to obtain the bioactive final surface composition it was started to replace the nitrogen by oxygen up to a proportion of 4 parts nitrogen and 1 part oxygen, which compose 10 percent of the total pressure of 10⁻³ hPa. The variation of the partial pressures of the reactive gases was done continuously or in fine steps in order to give the total coating the desired nanometric variation with regard to stoichiometry. Throughout high purity gases of grade 6.0 were used.

The same procedure was applied when using high intensity re-active radio-frequency magnetron sputtering.

The coating obtainable by the method according to the present invention is further illustrated in Fig. 1. Area 1 in Fig. 1 is a sub layer due to ion implantation. This layer was formed when the primary particle energy was at least 50 eV. Its thickness was between one and two nanometres (nm). This penetration layer and the subsequent layer (area) 2 in Fig. 1 are essential for the good adhesion and fracture toughness of the layer, since it delivers a very high critical stress intensity factor Kic of above 200, thus stopping any potential crack and gives protection of the body tissue to potentially harmful contact with pure metal ions such as nickel, contained in any austenitic stainless steel. Area 3 represents an intermediate area with controlled increase of the nitrogen partial pressure in the coating reactor. The argon (Ar) partial pressure was decreased accordingly. The partial pressure of the reactive gas was in function with the reactor geometry, the metal beam (Ti, Zr or an alloy of these, but essentially titanium with 20 % zirconium) ionization was chosen by the person skilled in the art accordingly. Area 4 was an intermediate non-stoichiometric stabilization region, with essentially constant Kic. Area 5a lead to the establishment of an important intermediate area 5b, composed of non-stoichiometric M₂N (double binary alloy nitride), again an important barrier for crack propagation due to its mixed covalent and ionic chemical bonding. The nitrogen partial pressure was further increased in areas 6 and 8, with a relaxation area 6 with 60 % Titanium, Zirconium or its alloy of essentially 80/20 to be stabilized at 50 % nitrogen, pure titanium nitride, area 9, known for its toughness and fatigue resistance from the tool making industry. Area 10 follows with the introduction of oxygen in appropriate partial pressure in order to achieve the medically active layer composed of, as chosen in the presented drawing but not exclusively, 50 % titanium, 40 % nitrogen and 10 % oxygen. The titanium can be replaced by zirconium or a titaniumzirconium alloy, essentially of 80 % titanium and 20 % zirconium. More generally, but not exclusively, most solid solutions of above mentioned elements can be applied. The preferred embodiment contains in general 80 % of titanium.

## Claims

1. A substrate comprising a single layer coating comprising an ion-implanted adhesion area (1, 2) located on the inner side of the layer, a cross-sectional area (3, 4, 5a, 5b, 6, 7, 8, 9) being **characterised by** a variation of its composition within its cross-section sandwiched between said adhesion area and an outermost area (10, 11), wherein
- the ion-implanted adhesion area being composed of titanium, zirconium, hafnium or alloys thereof (1, 2) has a thickness of 0.1 to 10 nm,
- the cross-sectional area (3, 4, 5a, 5b, 6, 7, 8, 9) comprises nitrides of titanium, zirconium, hafnium or alloys thereof having the general formula M_{X}N_{Y} with X+Y being 1 and X<1, 0<Y<1, with M being titanium, zirconium, hafnium or alloys thereof and with N being nitrogen, and having a thickness of 2 to 30 nm, preferably of 2 to 20 nm, in which is contained an area (5b) of a composition of M_{X}N_{Y1} with Y1 being a number between 0.9 and 1.1 having throughout its cross-section a nitrogen gradient between 1 and 100 %, and
- the outermost area (10, 11) comprises oxynitride of the general formula MN_{Z}O_{W}, wherein Z+W is 1, with Z varying from 0.2 to 0.9, with M being a solid transition metal of group IV B, V B or VI B of the periodic table of the elements or a solid solution thereof, with N being nitrogen and with O being oxygen, and has a thickness of 1 to 200 nm, preferably 2 to 50 nm.

2. Substrate according to claim 1, **characterised in that** the titanium alloy comprises 20 % zirconium and/or hafnium.

3. Substrate according to claim 1 or 2, **characterised in that** the outermost area comprises nitrides or oxynitrides of titanium, zirconium and hafnium or mixtures thereof.

4. Substrate according to claim 3, **characterised in that** titanium oxynitride has the formula TiN_{A}O_{B}, wherein A+B is 1, with A varying from 0.1 to 0.9, preferably from 0.2 to 0.5, particularly being 0.25.

5. Substrate according to any one of claims 1 to 4, **characterised in that** the single layer coating has a thickness of 10 to 200 nm, preferably 20 to 100 nm, more preferably 30 to 60 nm, in particular 50 nm.

6. Substrate according to any one of claims 1 to 5, **characterised in that** the adhesion area has a thickness of 1 to 10 nm, preferably of 2 to 5 nm.

7. Substrate according to any one of claims 1 to 6, **characterised in that** the substrate material is selected from the group of steel, preferably stainless austenitic steel, nitinol, titanium or alloys thereof.

8. Substrate according to any one of claims 1 to 7, **characterised in that** the substrate is an orthopaedic implant, preferably a hip, knee, shoulder or elbow implant, or a vascular implant, preferably a vascular graft or a vascular stent.

9. A method for producing a coating layer of a substrate according to any one of claims 1 to 8 using a deposition apparatus comprising a vacuum chamber and a power supply having controllable supply parameters, in which the substrate is enclosed in the vacuum chamber and subjected to bombardment by electrically-excited ions by electrical excitation provided by the power supply, said method comprising the steps of:
a) providing an inert gas and a target comprising titanium, chromium, zirconium and hafnium or alloys thereof, in the vacuum chamber,
b) performing a physical vapour deposition process for coating the surface of the substrate with an adhesion layer comprising titanium, chromium, zirconium and hafnium or alloys thereof, and
c) introducing into the vacuum chamber a gas mixture comprising a varying amount of nitrogen and/or oxygen while performing the physical vapour deposition process, wherein step c) is performed in part with a target consisting of titanium, zirconium or alloys thereof in an atmosphere containing 10 to 30% N₂.

10. Method according to claim 9, **characterised in that** the physical vapour process is selected from the group of sputter deposition, cathodic arc deposition, reactive filtered arc plasma deposition, reactive filtered arc plasma deposition with Wehnelt plasma confinement, reactive filtered arc plasma deposition with Wehnelt plasma confinement and an electrostatic or electromagnetic scanning device and high intensity radio frequency magnetron sputtering.

11. Method according to claim 9 or 10, **characterised in that** the amount of nitrogen and/or oxygen in the gas mixture of step c) is stepwise or gradually increased from 0 to 30 %, preferably from 0 to 20 %, more preferably from 0 to 15 %, while performing the physical vapour deposition process to obtain a coating layer with a thickness of 10 to 200 nm, preferably 20 to 100 nm, more preferably 30 to 60 nm, in particular 50 nm.

12. Method according to any one of claims 9 to 11, **characterised in that** the amount of nitrogen in the gas mixture is stepwise or gradually increased to 30 %, preferably 25 %, more preferably to 20 %, followed by a stepwise or gradual increase of oxygen in the gas mixture to 20 %, preferably 10 %.

13. Method according to any one of claims 9 to 12, **characterised in that** the substrate is an implant which is preferably an orthopaedic implant, preferably a hip, knee, shoulder or elbow implant, or a vascular implant, preferably a vascular graft or a vascular stent.

## Patentansprüche

1. Substrat, umfassend eine einlagige Beschichtung, welche eine Ionen-implantierte Haftfläche (1, 2) umfasst, die an der Innenseite der Lage lokalisiert ist, wobei eine Querschnittsfläche (3, 4, 5a, 5b, 6, 7, 8, 9) durch eine Variation ihrer Zusammensetzung innerhalb ihres zwischen dieser Haftfläche und einer äußersten Fläche (10, 11) eingeschlossenen Querschnitts gekennzeichnet ist, wobei
- die Ionen-implantierte Haftfläche, die sich aus Titan, Zirkonium, Hafnium oder Legierungen daraus (1, 2) zusammensetzt, eine Dicke von 0,1 bis 10 nm aufweist,
- die Querschnittsfläche (3, 4, 5a, 5b, 6, 7, 8, 9) Nitride von Titan, Zirkonium, Hafnium oder Legierungen daraus mit der allgemeinen Formel M_{X}N_{Y}, wobei X+Y gleich 1 ist und X<1, 0<Y<1 ist, worin M Titan, Zirkonium, Hafnium oder Legierungen daraus sind und worin N Stickstoff ist, und mit einer Dicke von 2 bis 30 nm, vorzugsweise 2 bis 20 nm, umfasst, worin eine Fläche (5b) von einer Zusammensetzung M_{X}N_{Y1}, worin Y1 eine Zahl zwischen 0,9 und 1,1 ist, enthalten ist, die durch ihren gesamten Querschnitt einen Stickstoffgradienten zwischen 1 und 100% aufweist, und
- die äußerste Fläche (10, 11) Oxinitrid der allgemeinen Formel MN_{z}O_{W}, worin Z+W gleich 1 ist, wobei Z von 0,2 bis 0,9 variiert, worin M ein festes Übergangsmetall der Gruppe IV B, V B oder VI B des Periodensystems der Elemente oder eine feste Lösung davon ist, worin N Stickstoff ist und worin O Sauerstoff ist, umfasst und eine Dicke von 1 bis 200 nm, vorzugsweise 2 bis 50 nm, aufweist.

2. Substrat gemäß Anspruch 1, **dadurch gekennzeichnet, dass** die Titanlegierung 20 % Zirkonium und/oder Hafnium umfasst.

3. Substrat gemäß Anspruch 1 oder 2, **dadurch gekennzeichnet, dass** die äußerste Fläche Nitride oder Oxinitride von Titan, Zirkonium und Hafnium oder Gemischen daraus umfasst.

4. Substrat gemäß Anspruch 3, **dadurch gekennzeichnet, dass** Titanoxinitrid die Formel TIN_{A}O_{B} aufweist, worin A+B gleich 1 ist, worin A von 0,1 bis 0,9, vorzugsweise von 0,2 bis 0,5, variiert und insbesondere 0,25 ist.

5. Substrat gemäß irgendeinem der Ansprüche 1 bis 4, **dadurch gekennzeichnet, dass** die einlagige Beschichtung eine Dicke von 10 bis 200 nm, vorzugsweise 20 bis 100 nm, mehr bevorzugt 30 bis 60 nm, insbesondere von 50 nm, aufweist.

6. Substrat gemäß irgendeinem der Ansprüche 1 bis 5, **dadurch gekennzeichnet, dass** die Haftfläche eine Dicke von 1 bis 10 nm, vorzugsweise von 2 bis 5 nm, aufweist.

7. Substrat gemäß irgendeinem der Ansprüche 1 bis 6, **dadurch gekennzeichnet, dass** das Substratmaterial ausgewählt ist aus der Gruppe von Stahl, vorzugsweise austenitischem Edelstahl, Nitinol, Titan oder Legierungen daraus.

8. Substrat gemäß irgendeinem der Ansprüche 1 bis 7, **dadurch gekennzeichnet, dass** das Substrat ein orthopädisches Implantat, vorzugsweise ein Hüft-, Knie-, Schulter- oder Ellbogenimplantat, oder ein Gefäßimplantat, vorzugsweise ein Gefäßtransplantat oder eine Gefäßprothese, ist.

9. Verfahren zur Herstellung einer Beschichtungslage auf einem Substrat gemäß irgendeinem der Ansprüche 1 bis 8 unter Verwendung eines Ablagerungsapparates, umfassend eine Vakuumkammer und eine Energiezufuhr mit kontrollierbaren Zufuhrparametern, wobei das Substrat in der Vakuumkammer eingeschlossen wird und einem Beschuss mit elektrisch angeregten Ionen mittels einer durch die Energiezufuhr bereitgestellten elektrischen Anregung ausgesetzt wird, welches Verfahren die folgenden Schritte umfasst:
a) Bereitstellen eines inerten Gases und eines Beschichtungswerkstoffs ("Target" = Zielscheibe), umfassend Titan, Chrom, Zirkonium und Hafnium oder Legierungen daraus, in der Vakuumkammer,
b) Durchführen eines physikalischen Dampfablagerungsverfahrens zur Beschichtung der Oberfläche des Substrats mit einer Haftschicht, umfassend Titan, Chrom, Zirkonium und Hafnium oder Legierungen daraus, und
c) Einbringen in die Vakuumkammer eines Gasgemischs, umfassend eine variierende Menge an Stickstoff und/oder Sauerstoff, während der Durchführung des physikalischen Dampfablagerungsverfahrens, wobei Schritt c) zum Teil mit einer Zielscheibe (Target), bestehend aus Titan, Zirkonium oder Legierungen daraus, in einer Atmosphäre, enthaltend 10 bis 30% N₂, vorgenommen wird.

10. Verfahren gemäß Anspruch 9, **dadurch gekennzeichnet, dass** das physikalische Aufdampfverfahren ausgewählt ist aus der Gruppe der Sputter-(Zerstäubungs-)-Ablagerung, Kathodenbogen-Ablagerung, reaktiven gefilterten Bogenplasma-Ablagerung, reaktiven gefilterten Bogenplasma-Ablagerung mit Wehnelt-Plasmaeinschluss, reaktiven gefilterten Bogenplasma-Ablagerung mit Wehnelt-Plasmaeinschluss und einer elektrostatischen oder elektromagnetischen Scanning-Vorrichtung und hochintensivem Radiofrequenz-Magnetron-Sputtering.

11. Verfahren gemäß Anspruch 9 oder 10, **dadurch gekennzeichnet, dass** die Menge an Stickstoff und/oder Sauerstoff in dem Gasgemisch von Schritt c) schrittweise oder graduell von 0 auf 30%, vorzugsweise von 0 auf 20%, mehr bevorzugt von 0 auf 15%, während der Durchführung des physikalischen Dampfablagerungsverfahrens erhöht wird, um eine Beschichtungslage mit einer Dicke von 10 bis 200 nm, vorzugsweise 20 bis 100 nm, mehr bevorzugt 30 bis 60 nm, insbesondere von 50 nm, zu erhalten.

12. Verfahren gemäß irgendeinem der Ansprüche 9 bis 11, **dadurch gekennzeichnet, dass** die Menge an Stickstoff in dem Gasgemisch schrittweise oder graduell auf 30%, vorzugsweise auf 25%, mehr bevorzugt auf 20% erhöht wird, gefolgt von einem schrittweisen oder graduellen Anstieg des Sauerstoffs in dem Gasgemisch auf 20%, vorzugsweise auf 10%.

13. Verfahren gemäß irgendeinem der Ansprüche 9 bis 12, **dadurch gekennzeichnet, dass** das Substrat ein Implantat ist, welches vorzugsweise ein orthopädisches Implantat, vorzugsweise ein Hüft-, Knie-, Schulter- oder Ellbogenimplantat, oder ein Gefäßimplantat, vorzugsweise ein Gefäßtransplantat oder eine Gefäßprothese, ist.

## Revendications

1. Substrat comprenant un revêtement à couche unique comprenant une zone d'adhérence à implantation d'ions (1, 2) située sur la face interne de la couche, une zone de section transversale (3, 4, 5a, 5b, 6, 7, 8, 9) étant **caractérisée par** une variation de sa composition dans sa section transversale intercalée entre ladite zone d'adhérence et une zone externe (10, 11), dans lequel
- la zone d'adhérence à implantation d'ions qui est composée de titane, de zirconium, d'hafnium ou d'alliages de ceux-ci (1, 2) a une épaisseur de 0,1 à 10 nm,
- la zone de section transversale (3, 4, 5a, 5b, 6, 7, 8, 9) comprend des nitrures de titane, de zirconium, d'hafnium ou des alliages de ceux-ci ayant la formule générale MₓN_{y}, où X + Y vaut 1 et X < 1, 0 < Y < 1, M étant le titane, le zirconium, l'hafnium ou des alliages de ceux-ci et N étant l'azote, et ayant une épaisseur de 2 à 30 nm, de préférence de 2 à 20 nm, dans laquelle est contenue une zone (5b) d'une composition de MₓN_{y1,} Y1 étant un nombre entre 0,9 et 1,1, ayant sur toute sa section transversale un gradient d'azote entre 1 et 100 %, et
- la zone externe (10, 11) comprend un oxynitrure de formule générale MN_{z}O_{w}, où Z + W vaut 1, Z variant de 0,2 à 0,9, M étant un métal de transition solide du groupe IV B, V B ou VI B du tableau périodique des éléments ou une solution solide de celui-ci, N étant l'azote et O étant l'oxygène, et a une épaisseur de 1 à 200 nm, de préférence de 2 à 50 nm.

2. Substrat selon la revendication 1, **caractérisé en ce que** l'alliage de titane comprend 20 % de zirconium et/ou d'hafnium.

3. Substrat selon la revendication 1 ou 2, **caractérisé en ce que** la zone externe comprend des nitrures ou des oxynitrures de titane, de zirconium et d'hafnium ou des mélanges de ceux-ci.

4. Substrat selon la revendication 3, **caractérisé en ce que** l'oxynitrure de titane a la formule TiN_{A}O_{B}, où A + B vaut 1, A variant de 0,1 à 0,9, de préférence de 0,2 à 0,5, valant en particulier 0,25.

5. Substrat selon l'une quelconque des revendications 1 à 4, **caractérisé en ce que** le revêtement à couche unique a une épaisseur de 10 à 200 nm, de préférence de 20 à 100 nm, de manière davantage préférée de 30 à 60 nm, en particulier de 50 nm.

6. Substrat selon l'une quelconque des revendications 1 à 5, **caractérisé en ce que** la zone d'adhérence a une épaisseur de 1 à 10 nm, de préférence de 2 à 5 nm.

7. Substrat selon l'une quelconque des revendications 1 à 6, **caractérisé en ce que** le matériau de substrat est choisi dans le groupe comprenant l'acier, de préférence l'acier inoxydable austénitique, le nitinol, le titane ou des alliages de ceux-ci.

8. Substrat selon l'une quelconque des revendications 1 à 7, **caractérisé en ce que** le substrat est un implant orthopédique, de préférence un implant de hanche, de genou, d'épaule ou de coude, ou un implant vasculaire, de préférence un greffon vasculaire ou un stent vasculaire.

9. Procédé de production d'une couche de revêtement d'un substrat selon l'une quelconque des revendications 1 à 8, en utilisant un appareil de dépôt comprenant une chambre à vide et une source d'énergie ayant des paramètres d'alimentation contrôlables, dans lequel le substrat est enfermé dans la chambre à vide et soumis à un bombardement par des ions excités électriquement par une excitation électrique fournie par la source d'énergie, ledit procédé comprenant les étapes de :
a) fourniture d'un gaz inerte et d'une cible comprenant le titane, le chrome, le zirconium et l'hafnium ou des alliages de ceux-ci, dans la chambre à vide,
b) réalisation d'un processus de dépôt physique en phase vapeur pour revêtir la surface du substrat avec une couche d'adhérence comprenant le titane, le chrome, le zirconium et l'hafnium ou des alliages de ceux-ci, et
c) introduction, dans la chambre à vide, d'un mélange de gaz comprenant une quantité variable d'azote et/ou d'oxygène tout en réalisant le processus de dépôt physique en phase vapeur, dans lequel l'étape c) est réalisée en partie avec une cible consistant en titane, zirconium ou des alliages de ceux-ci, dans une atmosphère contenant 10 à 30 % de N₂.

10. Procédé selon la revendication 9, **caractérisé en ce que** le processus de dépôt physique en phase vapeur est choisi dans le groupe comprenant le dépôt par pulvérisation, le dépôt à arc cathodique, le dépôt par plasma à arc filtré réactif, le dépôt par plasma à arc filtré réactif avec un confinement de plasma Wehnelt, le dépôt par plasma à arc réactif filtré avec un confinement de plasma Wehnelt et un dispositif de balayage électrostatique ou électromagnétique et la pulvérisation au magnétron à radiofréquences de haute intensité.

11. Procédé selon la revendication 9 ou 10, **caractérisé en ce que** la quantité d'azote et/ou d'oxygène dans le mélange de gaz de l'étape c) est augmentée par étapes ou progressivement de 0 à 30 %, de préférence de 0 à 20 %, de manière davantage préférée de 0 à 15 %, tout en réalisant le processus de dépôt physique en phase vapeur pour obtenir une couche de revêtement présentant une épaisseur de 10 à 200 nm, de préférence de 20 à 100 nm, de manière davantage préférée de 30 à 60 nm, en particulier de 50 nm.

12. Procédé selon l'une quelconque des revendications 9 à 11, **caractérisé en ce que** la quantité d'azote dans le mélange de gaz est augmentée par étapes ou progressivement à 30 %, de préférence à 25 %, de manière davantage préférée à 20 %, suivi d'une augmentation par étapes ou progressive de l'oxygène dans le mélange de gaz à 20 %, de préférence à 10 %.

13. Procédé selon l'une quelconque des revendications 9 à 12, **caractérisé en ce que** le substrat est un implant qui est de préférence un implant orthopédique, de préférence un implant de hanche, de genou, d'épaule ou de coude, ou un implant vasculaire, de préférence un greffon vasculaire ou un stent vasculaire.
